(19) Europäisches Patentamt
European Patent Office
Office européen des brevets

(11) **EP 3 787 708 B1**

(12) **EUROPÄISCHE PATENTSCHRIFT**

(45) Veröffentlichungstag und Bekanntmachung des Hinweises auf die Patenterteilung:
**24.11.2021 Patentblatt 2021/47**

(21) Anmeldenummer: **19727927.6**

(22) Anmeldetag: **02.05.2019**

(51) Int Cl.:
*A61M 3/02* (2006.01)          *A61B 1/015* (2006.01)
*A61B 17/02* (2006.01)

(86) Internationale Anmeldenummer:
**PCT/DE2019/000118**

(87) Internationale Veröffentlichungsnummer:
**WO 2019/210894 (07.11.2019 Gazette 2019/45)**

(54) **VERFAHREN ZUR BESTIMMUNG DES HÖHENUNTERSCHIEDES ZWISCHEN PATIENT (OPERATIONSHÖHE) UND EINER FLUID-FÖRDERPUMPE**

METHOD FOR ASCERTAINING THE VERTICAL DISTANCE BETWEEN A PATIENT (LEVEL OF SURGICAL INTERVENTION) AND A FLUID DELIVERY PUMP

PROCÉDÉ POUR DÉTERMINER LA DIFFÉRENCE DE HAUTEUR ENTRE UN PATIENT (HAUTEUR D'INTERVENTION CHIRURGICALE) ET UNE POMPE D'ALIMENTATION EN FLUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.05.2018 DE 102018003519**

(43) Veröffentlichungstag der Anmeldung:
**10.03.2021 Patentblatt 2021/10**

(73) Patentinhaber: **W.O.M. World of Medicine GmbH**
**10587 Berlin (DE)**

(72) Erfinder:
• **CHRISTMANN, Thomas**
  **13187 Berlin (DE)**
• **CARSTENS, Jan Hendrik**
  **13086 Berlin (DE)**
• **CAPPIUS, Hans-Joachim**
  **12157 Berlin (DE)**

(74) Vertreter: **Jungblut & Seuss**
**Patentanwälte**
**Max-Dohrn-Strasse 10**
**10589 Berlin (DE)**

(56) Entgegenhaltungen:
**US-A- 4 795 424          US-A1- 2015 290 387**
**US-A1- 2017 209 639      US-A1- 2017 348 137**

EP 3 787 708 B1

**Beschreibung**

**Erfindungsgegenstand**

Die Erfindung betrifft ein Verfahren zur Ermittlung der Höhendifferenz zwischen einer medizinischen Fluidpumpe und der Körperhöhle eines Patienten zur Korrektur des am Auslaufende vorhandenen Fluiddruckes.

**Stand der Technik**

Es ist bekannt, im Rahmen der minimal-invasiven Chirurgie (MIC) Fluide (z.B. wässrige Spüllösungen) in Körperhöhlen zu pumpen, um die Körperhöhle aufzudehnen. Hierfür muss der Druck groß genug sein, um das Gewebe zu dehnen, darf aber nicht zu groß werden, um das Gewebe nicht zu schädigen. Die Messung des Druckes in der Körperhöhle ist somit von hoher Bedeutung. Dabei wird aus verschiedenen Gründen (z.B. Platzbedarf und Kontaminationsgefahr) kein Drucksensor in der Körperhöhle platziert. Es muss daher auf das Ergebnis der Druckmessung in der Pumpe zurückgegriffen werden. Der in der Pumpe gemessene Druck ist jedoch nur dann gleich dem Druck in der Körperhöhle, wenn der Drucksensor der Pumpe auf der gleichen Höhe ist, wie die Körperhöhle. Wenn es eine Höhendifferenz zwischen Drucksensor und Körperhöhle gibt, dann weicht der vom Drucksensor gemessene Wert vom tatsächlichen Druckwert in der Körperhöhle ab, wobei die Abweichung proportional zur Höhendifferenz ist. Diese eventuelle Messabweichung in Folge der Positionierung von Pumpe relativ zum Patienten bleibt bei Pumpen des Standes der Technik unberücksichtigt. Es besteht daher Bedarf an einem Verfahren welches die Höhendifferenz zwischen Pumpe und Körperhöhle des Patienten vor Operationsbeginn ermittelt und die gemessenen Druckwerte bei Bedarf korrigiert.

Die Druckschrift US 2015/0290387A1 offenbart eine Vorrichtung zur Insufflation mit einer blutdruckabhängigen Drucksteuerung. Die Druckschrift US 2008/0243054A1 offenbart eine Vorrichtung zur Durchspülung einer Körperhöhle, wobei der Druck und Fluss der Spülflüssigkeit in Abhängigkeit vom Druck in der durchspülten Körperhöhle geregelt wird. Eine Ermittlung der Höhendifferenz zwischen Pumpe und Körperhöhle des Patienten vor Operationsbeginn wird in beiden Druckschriften nicht vorgenommen.

Als Voraussetzungen für die Anwendung der erfindungsgemäßen Methode zur Höhenermittlung, die für eine bestimmte Situation im medizinischen Behandlungsumfeld vorgesehen ist, jedoch keine Behandlungsmethode des menschlichen Körpers an sich darstellt, sind Randbedingungen zu erfüllen, die im Folgenden beschrieben werden. Die Situation in der die erfindungsgemäße Methode zum Einsatz kommt, ist wie folgt: An einer Fluid-Förderpumpe (5) (im Folgenden nur Pumpe genannt) wird ein gewünschter Solldruck vorgegeben, welcher beispielsweise mittels Fluid zur Aufdehnung in einer Kavität eines Patienten (10) aufgebaut werden soll. Eine solche Kavität kann eine natürliche Kavität wie z.B. ein Gelenkzwischenraum oder der Bauchraum sein oder eine künstlich geschaffene Körperhöhle, die zu diagnostischen und/oder therapeutischen Zwecken aufgedehnt wird. Die Aufdehnung geschieht mit einem vorbestimmten Solldruck, der durch Förderung des Fluides in die Kavität erreicht und aufrechterhalten wird. Zur Förderung eines solchen Fluids aus einem Vorratsbehälter (1) in die Kavität wird erfindungsgemäß als Pumpe (5) eine Peristaltikpumpe genutzt.

Als Messvorrichtung zur Druckmessung befindet sich an der Pumpe (5) mindestens ein Drucksensor (6), der den Förderdruck $P_{drv}$ ermittelt. Im Falle, dass das Auslaufende der Fluidleitung (7) bzw. der Operationsbereich (gleichbedeutend mit der durch Fluid aufgedehnten Kavität) nicht in derselben Höhenlage wie die Pumpe (5) ist, ändert der hydrostatische Druck ($P_{hyd}$) des Fluids im Schlauchabschnitt (7) den Kavitätsdruck $P_{cav}$ bzw. wirkt auf den vom Drucksensor gemessenen Wert $P_{drv}$. Der Wert des Drucksensors kann somit nicht ohne weitere Kompensation zur Regelung des Kavitätsdruckes $P_{cav}$ benutzt werden. Der Kavitätsdruck $P_{cav}$ ist die zu regelnde Zielgröße, die auf einem vorgegebenen Solldruck gehalten werden soll.

Die Ausgangslage, in der die erfindungsgemäße Methode angewendet werden soll, kann in verschiedenen Szenarien erfolgen, betreffend den Höhenunterschied zwischen Fördervorrichtung und Auslaufende sowie betreffend die Situation am Auslaufende. Es wird davon ausgegangen, dass sich die Szenarien in Bezug auf die Höhen nicht ändern über die Anwendungsdauer, d.h. von der ersten Anwendung der erfindungsgemäßen Methode bis die Fluidverbindung zum Vorratsbehälter verworfen wird und ein neuer Schlauch (3, 7) in die Fluid-Förderpumpe (5) eingelegt wird.

Ziel der erfindungsgemäßen Lösung ist die Ermittlung der Patientenhöhe $h_{pat}$ (Patient (10) kann höher oder tiefer als die Pumpe (5) gelagert sein), wodurch der durch Einsatz des Drucksensors (6) geregelte Sollwert entsprechend den konkreten Gegebenheiten kompensiert werden kann. Ohne diese Kompensation kann der Druck $P_{cav}$ in der Kavität des Patienten (10) nicht korrekt auf den Sollwert eingeregelt werden.

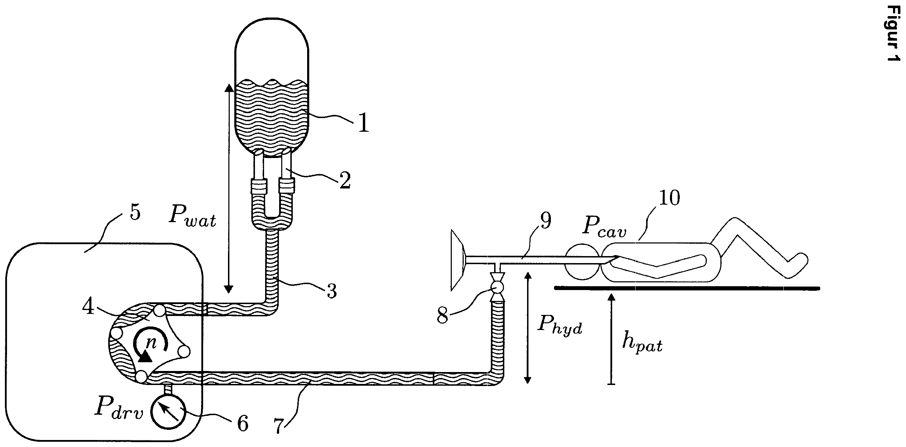

Im Falle, dass der *Patient (10) höher als die Pumpe* (5) gelagert wird **(Figur 1)**, wirkt sich der hydrostatische Druck ($P_{hyd}$) des Fluides im Schlauchabschnitt (7) auf den Sensor (6) als Offsetfehler aus und kann gemäß Stand der Technik abgezogen werden. Es wird erfindungsgemäß für die Druck- bzw. Höhenermittlung eine alternative Lösung beschrieben. Hierbei ist der Druck in cm *Wassersäule* in den zu ermittelnden Höhenunterschied in cm überführbar.

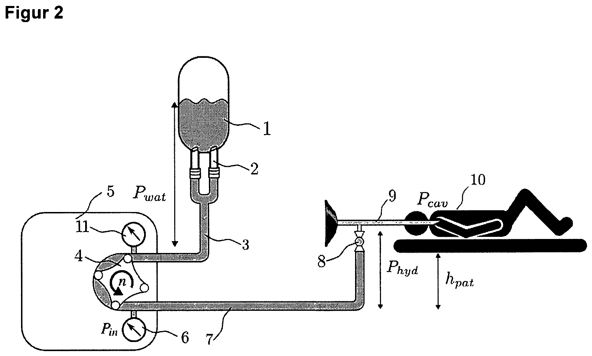

Im Falle, dass der *Patient (10) tiefer als die Pumpe* (5) gelagert ist **(Figur 2)**, wirkt sich der hydrostatische Druck ($P_{hyd}$) des Fluides im Schlauchabschnitt (7) als Offset auf den Kavitätsdruck $P_{cav}$ im Patienten (10) aus. Dieser Fall kann ebenfalls mit einem Drucksensor (6) erfasst werden, solange dieser Drucksensor (6) in Bezug auf den Umgebungsdruck negative Druckwerte ermitteln kann. Wird ein Drucksensor (6) genutzt, der nur positive Druckwerte ermitteln kann, kann der obige genannte Fall nicht direkt messtechnisch ermittelt werden. Es wird erfindungsgemäß für die Druck- bzw. Höhenermittlung eine alternative Lösung beschrieben.

In Fluid-Förderpumpen entsprechend dem Stand der Technik wird eine Höhendifferenzkorrektur durch Vorgabe/Eingabe eines Wertes ($h_{pat}$) in die Systemeinstellungen der Fluid-Förderpumpe (5) angeboten. Bei dieser Höheneinstellung können entsprechend dem Stand der Technik nur positive Höhen eingegeben werden. Dies deckt nur den Fall ab, dass der *Operationsbereich/Patient* (10) *höher liegt als die Pumpe* (5). Auf Grundlage dieses Wertes, wird der hydrostatische Druck ($P_{hyd}$) am Sensor (6) kompensiert (Offsettkompensation), also vom gemessenen Wert $P_{drv}$ abgezogen. Dieses Verfahren wird auch in den Patentanmeldungen WO 2005/089832 A2 und WO 2005/042065 A2 ausgeführt. Weiterer relevanter Stand der Technik ist beispielsweise in den Dokumenten US2017/348137 A1, US2017/209639 A1 und US4795424 A offenbart.

Ein Verfahren welches auf Basis mehrerer Messungen eine Patientenhöhe $h_{pat}$ ermittelt ist soweit nicht bekannt.

**Erfindungsgemäße Lösung**

Da im Kontext der Geräteanwendung zwar dem Anwender ein bestimmtes Verhalten auferlegt werden kann, dieses aber nicht vom Hersteller überprüfbar ist und der Hersteller für die sichere Anwendung verantwortlich ist, wird im Folgenden ein für alle denkbaren Szenarien wirksames, erfindungsgemäßes technisches Verfahren ausgeführt. Zunächst sind die Varianten der Szenarien beschrieben:
Grundsätzlich ist die Lage des Patienten (10) - damit ist die auszudehnende Körperhöhle gemeint - oberhalb oder unterhalb des Förderrollenrades (4) zu unterscheiden.

Als zusätzlicher Fall muss ermittelt werden, ob die *Verbindung* vom Sensor (6) über Schlauchabschnitt (7), Konnektor mit Auslaufventil (8) und ein Instrument (9) *offen* zur Raumatmosphäre ist. Im Fall, dass das *Instrument (9) in einer geschlossenen Kavität des Patienten* (10) *eingeführt* wurde, muss der hydraulische Druck ($P_{hyd}$) vom Druck in der Kavität $P_{cav}$ unterschieden werden.

Zur Ermittlung des hydraulischen Drucks bzw. Patientenhöhe ($h_{pat}$), kann erfindungsgemäß ein Verfahren auf Basis der Messung von Druckwerten genutzt werden.

Hierzu muss zunächst geprüft werden wie viele Drucksensoren welcher Art in der Pumpe verbaut sind. In der Regel ist ein Drucksensor in Förderrichtung nach der Pumpe vorhanden. Dieser kann uni- oder bidirektionale Messungen ermöglichen, also entweder nur positive Druckwerte anzeigen oder positive und negative. Manche Pumpenmodelle weisen einen zusätzlichen Drucksensor zwischen dem Vorratsbehälter (1) und der Pumpe (5) auf.

Die erfindungsgemäße Lösung für die Ermittlung der Patientenhöhe $h_{pat}$, die einen hydrostatischen Druck ($P_{hyd}$) bewirkt, muss zwischen zwei Anwendungsfällen unterscheiden:

I) Der Patient (Operationsbereich) (10) liegt höher als der Drucksensor (6) der Pumpe (5) ($h_{pat}>0$).

II) Der Patient (Operationsbereich) (10) liegt tiefer als der Drucksensor (6) der Pumpe (5) ($h_{pat}<0$).

Hierbei wird davon ausgegangen, dass zwischen Drucksensor (6) und hydraulisch wirksamer Höhe des Förderrollenrades (4) keine relevante Höhendifferenz ist. Ein Wert für 'keine relevante Höhendifferenz' ist $\leq$ 3 cm Wassersäule.

Ferner gibt es unterschiedliche Prozedurfälle:

a) Ein genutztes Instrument (9) ist außerhalb des Patienten (10) bzw. der Kavität des Operationsbereichs und das Auslaufende offen zur Raumatmosphäre bzw. zum Umgebungsdruck.

b) Das genutzte Instrument (9) wurde bereits in die Kavität des Operationsbereichs eingeführt und am Auslaufende liegt ein (erhöhter und unbekannter) Kavitätsdruck $P_{cav}$ an.

c) Das in der Fluidverbindung vorhandene Auslaufventil (8) ist verschlossen bzw. der Schlauchabschnitt (7) ist abgeklemmt.

Zur Lösung dieses Problems lehrt die vorliegende Patentanmeldung die Verfahrensvarianten gemäß der unabhängigen Ansprüche 1 - 4. Erfindungsgemäße Weiterentwicklungen werden durch die abhängigen Ansprüche 5-7 gelehrt.

Ein zentrales Element der vorliegenden Erfindung ist die Ermittlung des Druckes in den Schlauchsystemen durch Bestimmung des Drehmomentes der Pumpe wobei das Drehmoment durch die Messung des Stromes bestimmt wird. Diese Ausführungsform der Erfindung wird im unabhängigen Anspruch 9 gelehrt.

**Variante 1:** Die einfachste Ausführungsform der vorliegenden Erfindung ist in Figur 2 dargestellt: Ein Vorratsbehälter (1) ist über eine Schlauchverbindung (3) verbunden mit einer erfindungsgemäßen Rollradpumpe (5). Die Förderpumpe ist über den Schlauch (7) mit einem medizinischen Instrument (9) verbunden, welches in die Körperhöhle eines Patienten (10) eingeführt ist. Bestimmt werden soll der hydrostatische Druck der Wassersäule in der Körperhöhle (10) des Patienten. Der Druck $P_{CAV}$ setzt sich zusammen aus den Teildrucken der Wassersäule vor der Förderpumpe $P_{WAT}$ und dem Druck der Wassersäule nach der Förderpumpe $P_{HYD}$. Der Druck $P_{WAT}$ hängt ab von der Höhe des Vorratsbehälters über der Pumpe, der Druckanteil $P_{HYD}$ hängt von der Höhe des Patienten $H_{PAT}$ über- oder unterhalb des Förderrades der Pumpe (5) ab. Ist die Patientenhöhe unterhalb des Förderrades, dann addieren sich die Drucke $P_{WAT}$ und $P_{HYD}$ zum Gesamtdruck in der Körperhöhle $P_{CAV}$. Liegt der Patient oberhalb der Ebene des Förderrades, dann reduziert sich der Druck $P_{WAT}$ um den Druck $P_{HYD}$. Die Ermittlung der Patientenhöhe $H_{PAT}$ ist daher durch Messung der Drucke $P_{WAT}$ und $P_{HYD}$ grundsätzlich möglich.

Im einfachsten Fall (siehe Figur 2) weist die Pumpe zwei Drucksensoren auf, nämlich einen Drucksensor (11) vor der Förderpumpe (5) und einen Drucksensor (6) nach der Förderpumpe (5). Falls es sich bei dem Drucksensor (6) um einen bi-direktionalen Drucksensor handelt, dann können beide Drücke direkt von den Drucksensoren (11) und (6) abgelesen werden. Aus diesen Druckwerten lässt sich die Patientenhöhe unmittelbar ermitteln.

In diesem Fall ist lediglich zu prüfen, ob der Förderschlauch (7) mit dem medizinischen Instrument 9 in die Körperhöhle eingeführt ist, und ob das Ventil (8) geöffnet ist. Der Teilprozess zu Feststellung, ob das medizinische Instrument in die Körperhöhle eingeführt ist, und ob das Ventil geöffnet ist, ist wie folgt durchführbar:

Entscheidend für die Feststellung ist die Kenntnis des Schlauchvolumens des Förderschlauches (7). Erfindungsgemäß wird vorgeschlagen, dieses Volumen vor Inbetriebnahme der Pumpe zu ermitteln und in der Pumpe zu speichern. Im klinischen Regelfall werden immer gleichartige Schlauchsets verwendet, sodass das Schlauchvolumen nur einmal bestimmt werden muss. In einer bevorzugten Ausführungsform, der Erfindung wird das Schlauchvolumen in einem Speicher abgelegt, der an dem Schlauchset befestigt ist, z. B. einem RFID-Transponder. Bei Einlegen des Schlauchsets in die Pumpe wird der RFID-Transponder ausgelesen und das Schlauchvolumen der Pumpe zur Verfügung gestellt. Alternativ kann die Übermittlung auch über andere elektrische/elektronische, magnetische oder optische (z. B. Barcode) Übertragungselemente erfolgen.

Nach Ablage des Schlauchvolumens in einem Speicher der Pumpe kann die Förderung beginnen. Durch Anzeige eines von null abweichenden Druckwertes des Drucksensors (6) wird der Pumpe angezeigt, dass die Schlauchbefüllung des Förderschlauches (7) beginnt. Da die geförderte Flüssigkeitsmenge proportional zu den Drehungen der Pumpe ist, kann das in den Förderschlauch (7) geförderte Flüssigkeitsvolumen präzise ermittelt werden.

Für den gewünschten Fall, dass das medizinische Instrument bereits in die Körperhöhle eingefüllt ist und das Ventil (8) geöffnet ist, zeigt der Drucksensor (6) einen praktisch konstanten Druck an, bis der Schlauch komplett gefüllt ist, d. h. das geförderte Volumen dem Schlauchvolumen entspricht. In der Praxis oszilliert der Druck durch die Peristaltik der Pumpe geringfügig um einen konstanten Wert (z.B. $\pm$ 3cm Wassersäule). Eine derartige geringfügige Oszillation um einen konstanten Wert wird für die hier beschriebenen Zwecke als konstant angesehen. Sobald die Flüssigkeit in die Körperhöhle gelangt, baut sich in Folge der gewünschten Aufdehnung der Körperhöhle langsam ein höherer Druck auf. Der gesuchte hydrostatische Druck $P_{HYD}$ ist in diesem Fall der Druck der gemessen wird, nachdem das Fördervolumen dem Schlauchvolumen entspricht.

Für den Fall, dass das Ventil geschlossen ist, ist die Förderpumpe (5) nicht in der Lage den gesamten Schlauch zu befüllen, weil die im Schlauch befindliche Luft nicht entweichen kann und daher komprimiert wird. Dieser Zustand des Systems zeigt sich dadurch, dass bereits vor Förderung eines Flüssigkeitsvolumens, welches dem Schlauchvolumen entspricht, ein signifikanter Druckanstieg erfolgt. In diesem Fall ist eine Messung der Patientenhöhe nicht möglich. Die Pumpe gibt in diesem Fall ein Warnsignal aus. Wenn das Ventil geöffnet wird, entspricht die Situation dem oben beschriebenen Fall.

Sollte das Ende des Förderschlauches bzw. des medizinischen Instrumentes sich nicht in der Körperhöhle des Patienten befinden, dann hängt der vom Drucksensor (6) gemessene Druck davon ab, auf welcher Höhe sich das Schlauchende befindet. Der Drucksensor zeigt nach Förderung eines Flüssigkeitsvolumens, welches dem Schlauchvolumen entspricht einen Druck, der sich bei weiterer Förderung nicht erhöht. Auch in diesem Fall wird von der Pumpe ein Alarmsignal ausgelöst. Nach Anschluss des Förderschlauches an medizinische Instrument bzw. nach Einführung des medizinischen Instrumentes in die Körperhöhle des Patienten stellt sich wieder der oben genannte Zustand ein, der wie ob messtechnisch erfasst werden kann.

**Variante 2:** Im Fall einer Pumpe, die nur einen einzigen Drucksensor (6) nach dem Förderrad aufweist (siehe Figur 1), ist der oben beschriebene Prozess wie folgt zu modifizieren:

Zur Bestimmung des Druckes $P_{WAT}$ wird bei angeschlossenem Vorratsbehälter zunächst Flüssigkeit gefördert bis der Drucksensor (6) einen Druck anzeigt. Diese Druckanzeige dient als Hinweis dafür, dass der Schlauch zumindest bis zur Position des Drucksensors (6) gefüllt ist. Zu diesem Zeitpunkt wird die Förderung gestoppt, das Förderrad (4) kommt zum Stillstand. Dann wird eine "Rückwärtsförderung" eingeleitet, d. h. das Rollenrad dreht sich in die entgegengesetzte Richtung. Bei dieser Rückwärtsförderung wird das Drehmoment des Motors ermittelt. Hierzu dienen Stromsensoren (12), die den Stromfluss bei der Rückwärtsförderung messen. Der Stromfluss ist dabei proportional zum Drehmoment. Das Drehmoment setzt sich dabei zusammen aus einem konstanten und einem variablen Anteil. Diese Konstante wird gebildet durch den Rollwiderstand der Pumpe (5) in Kombination mit den Materialeigenschaften des Schlauches (3,7). Bei konstanten Schlauchmaterialien, wie sie im klinischen Betrieb verwendet werden, kann hier von einer Konstanz ausgegangen werden. Die zweite Komponente des Drehmomentes besteht aus dem hydrostatischen Druck $P_{WAT}$ gegen die Pumpe (5) fördern muss. Bei bekannten Pumpen und Schlauchmaterialien kann daher der Druck $P_{WAT}$ durch Messung des Stromflusses mittels der Stromsensoren (12) bestimmt werden.

Nach dieser Bestimmung des Druckanteiles $P_{WAT}$ kann das Verfahren wie weiter oben (Variante 1) beschrieben durchgeführt werden, sofern der Drucksensor (6) wie eingangs beschrieben ein bidirektionaler Drucksensor ist.

**Variante 3:** Für den Fall, dass der Drucksensor (6) ein unidirektionaler Drucksensor ist, der daher nur positive Drucke, nicht aber negative Drücke messen kann, müssen die oben dargestellten Verfahren wie folgt modifiziert werden: Zunächst wird der Druck $P_{WAT}$ wie in Variante 1 oder Variante 2 bestimmt.

Anschließend wird durch das Rollenrad (4) ein Flüssigkeitsvolumen gefördert, welches dem Schlauchvolumen entspricht. Wie in Variante 1 beschrieben, wird dabei festgestellt, ob das medizinische Instrument sich in der Körperhöhle befindet, und ob das Ventil (8) geöffnet ist. Wenn das Ventil (8) geöffnet ist und sich das medizinische Instrument (9) in der Körperhöhle (10) befindet, ein Volumen gefördert wurde, welches dem Schlauchvolumen entspricht und der Drucksensor einen positiven Druckwert anzeigt, dann entspricht dieser Druckwert dem Druck $P_{HYD}$. Die Körperhöhle liegt somit oberhalb des Förderrades und aus dem gemessenen Druck kann die Patientenhöhe oberhalb des Förderrades unmittelbar bestimmt werden. Zeigt der Drucksensor (6) jedoch keinen Druck an (Druck gleich Null), dann liegt der Patient entweder genau auf Höhe der Förderpumpe oder darunter. In diesem Fall kann der Druckwert $P_{HYD}$ wie folgt ermittelt werden:

Nach Förderung eines Flüssigkeitsvolumens, welches dem Schlauchvolumen entspricht, wird die Förderpumpe gestoppt und es erfolgt eine "Rückwärtsförderung" durch Rückwärtsdrehung des Förderrades. Analog zur oben beschriebenen Variante 2 wird mittels der Stromsensoren (12) der Stromfluss gemessen. Der Stromfluss wiederum ist proportional zum Drehmoment (zuzüglich der oben beschriebenen Konstante), welches proportional zum Gesamtdruck ist. In diesem Fall zeigt das Drehmoment den Druck der gesamten Flüssigkeitssäule, d. h. $P_{WAT} + P_{HYD}$, an. Durch Subtraktion des bereits bekannten Druckes $P_{WAT}$ vom Gesamtdruck kann der Druck $P_{HYD}$ ermittelt werden, der wiederum die Höhe der Körperhöhle des Patienten unterhalb der Förderpumpe angibt.

Der besondere Vorteil des erfindungsgemäßen Verfahrens besteht darin, dass die zur präzisen Messung der Patientenhöhe erforderlichen Daten schnell und präzise ermittelt werden können. Es können dabei auf einfache Weise ermittelt werden, ob das Förderrad der Pumpe in gleicher Höhe liegt, wie die Körperhöhle des Patienten, oder ob signifikante Abweichungen von der Wunschposition gegeben sind. Gleichzeitig werden Warnhinweise ausgegeben, falls Fehlbedienungen vorliegen (z.B. Ventil geschlossen oder Schlauch nicht an das medizinische Instrument angeschlossen).

**Detaillierte Ausführung der Erfindung**

Die erfindungsgemäße Lösung für die Ermittlung der Patientenhöhe $h_{pat}$, die einen hydrostatischen Druck ($P_{hyd}$) bewirkt, muss zwischen zwei Anwendungsfällen unterscheiden:

I) Der Patient (Operationsbereich) (10) liegt höher als der Drucksensor (6) der Pumpe (5) ($h_{pat} > 0$).

II) Der Patient (Operationsbereich) (10) liegt tiefer als der Drucksensor (6) der Pumpe (5) ($h_{pat} < 0$).

Hierbei wird davon ausgegangen, dass zwischen Drucksensor (6) und hydraulisch wirksamer Höhe des Förderrollenrades (4) keine relevante Höhendifferenz ist. Ein Wert für 'keine relevante Höhendifferenz' ist $\leq$ 3 cm Wassersäule.

Ferner gibt es unterschiedliche Prozedurfälle:

a) Ein genutztes Instrument (9) ist außerhalb des Patienten (10) bzw. der Kavität des Operationsbereichs und das Auslaufende offen zur Raumatmosphäre bzw. zum Umgebungsdruck.

b) Das genutzte Instrument (9) wurde bereits in die Kavität des Operationsbereichs eingeführt und am Auslaufende liegt ein (erhöhter und unbekannter) Kavitätsdruck $P_{cav}$ an.

c) Das in der Fluidverbindung vorhandene Auslaufventil (8) ist verschlossen bzw. der Schlauchabschnitt (7) ist abgeklemmt.

Im **Fall I), a)** wird ein Fluid von der Pumpe (5) aus dem Vorratsbehälter (1) gefördert um den Schlauch (3, 7) und das Instrument (9) mit dem Fluid zu füllen. Der Druck am Sensor (6) erreicht einen stationären Wert, d.h. er ändert sich im Mittelwert nur im unteren einstelligen Prozentbereich, wenn der Schlauch (3, 7) und das Instrument (9) gefüllt sind. In diesem Fall wird die Pumpe (5) gestoppt. Der nach dem Stopp der Pumpe (5) gemessene stationäre Druckwert ($P_{drv}$) entspricht dem hydrostatischen Druck ($P_{hyd}$). Durch den statischen Abzug des Wertes $P_{hyd}$ vom Sensorwert $P_{drv}$ wird der hydrostatische Druck ($P_{hyd}$) somit kompensiert.

Im **Fall II), a)** wird ein Fluid von der Pumpe (5) aus dem Vorratsbehälter (1) gefördert um den Schlauch (3, 7) und das Instrument (9) zu füllen. Der Druck am Sensor (6) erreicht einen stationären Wert, wenn der Schlauch (3, 7) und das Instrument (9) gefüllt sind. In diesem Fall wird die Pumpe (5) gestoppt. Der gemessene stationäre Druckwert ($P_{drv}$) entspricht dem hydrostatischen Druck ($P_{hyd}$), wenn ein **bidirektionaler Drucksensor** genutzt wird, der relativ zum Umgebungsdruck positive und negative Drücke messen kann. In diesem Fall kann der statische Wert $P_{hyd}$ zum Sensorwert $P_{drv}$ addiert werden um den hydrostatische Druck ($P_{hyd}$) in der Kavität zu bestimmen und den Messfehler zu kompensieren.

Um im **Fall II), a)** den hydrostatischen Druck ($P_{hyd}$) mittels **unidirektionalem Sensor** zu ermitteln, muss das Verfahren erweitert werden. Ein unidirektionaler Sensor kann nur über dem Umgebungsdruck liegende, also positive Werte messen. Nachdem der Schlauch (3, 7) und das Instrument (9) mit einer Flüssigkeit gefüllt sind und der Drucksensor (6) einen stationären Messwert ermittelt, wird die Pumpe (5) gestoppt. Im Anschluss wird die Pumpe (5) in der entgegengesetzten Förderrichtung betrieben und das Fluid im Schlauchabschnitt (7) ansaugt. Mittels Drehmomentenmessung des Pumpenmotors kann auf die notwendige Kraft bzw. unter Berücksichtigung des Schlauchinnendurchmessers den Ansaugdruck ($P_m$) an der Pumpe (5) geschlossen werden. Hierbei gilt Drehmoment $\sim$ Kraft $\sim$ Druck. Die Ermittlung des Drehmomentes kann über die Strommessung des Motors vom Förderrollenrad (4) der Pumpe (5) erfolgen. Wenn der notwendige Ansaugdruck bekannt ist, bei dem das Fluid aus dem Schlauch (3, 7) und Instrument (9) zurückgefördert wird, kann damit der hydrostatische Druck ($P_{hyd}$) berechnet werden. Hierbei bedarf es dem Vorwissen, wie hoch der hydrostatische Druck $P_{wat}$ vom Vorratsbehälter (1) zu Pumpenhöhe ist. Die Berechnung ergibt sich zu:

$$P_m - P_{wat} = P_{hyd}.$$

Wenn der hydrostatische Druck $P_{wat}$ des Vorratsbehälters (1) messtechnisch bestimmt werden soll, gibt es zwei Möglichkeiten: Zum einem die Nutzung eines zweiten Drucksensors (11) zwischen Förderrollenrad (4) und Vorratsbehälter (1). Zum anderen eine messtechnische Ermittlung des Drucks $P_{wat}$ über Drehmomentmessung der Fluid-Förderpumpe (5). Im zweiten Fall bedarf es einem initialen Messablauf, bevor der hydrostatische Druck ($P_{hyd}$) im Schlauchabschnitt (7) und Instrument (9) ermittelt werden kann. Die Pumpe (5) wird mit Fluid-entleerten Schläuchen gestartet, um das Fluid aus dem Vorratsbehälter (1) in den Schlauchabschnitt (3) zu fördern. In dem Moment, wo das Fluid das Förderrollenrad (4) bzw. den Ort des Drucksensors (6) erreicht und Wasser in den Schlauchabschnitt (7) zwischen Pumpe (5) und Patient (10) fördert, wird am Drucksensor (6) ein Druckanstieg ermittelt. Die Pumpe (5) wird gestoppt und die Förderrichtung geändert (Pumpe (5) fördert das Fluid zurück zum Vorratsbehälter (1), im Schlauchabschnitt (7) zwischen Pumpe (5) und Patient (10) ist keine signifikante Menge an Fluid). Das notwendige Motormoment der Pumpe (5), um das Fluid aus dem Schlauchabschnitt (7) in den Schlauchabschnitt (3) zwischen Vorratsbehälter (1) und Pumpe (5) zu fördern, ist proportional dem Druck $P_{wat}$ und der Proportionalitätsfaktor kann vorab durch Messung für den eingesetzten Schlauchinnendurchmesser des Schlauchabschnittes (3) ermittelt werden. Das Vorgehen dazu wäre eine vorherige Vermessung der Zusammenhänge und bei Inbetriebnahme der Pumpe (5) eine Zuordnung des Proportionalitätsfaktors je nach erkanntem Schlauchset. Die Erkennung und Datenübermittelung kann dabei z.B. mittels eines RFID-Chips im Schlauchset erfolgen.

In einer alternativen Ausführungsform kann aus Schlauchinnendurchmesser bzw. der daraus berechneten Schlauchquerschnittfläche des Schlauchabschnittes (3) und Motordrehmoment der Druck $P_{wat}$ und damit die Höhe zwischen Förderrollenrad (4) und dem Fluidspiegel im Vorratsbehälter (1) mathematisch bestimmt werden:

$$P_{wat} = \frac{Motordrehmoment}{Radius\ des\ Förderrollenrades\ (4) \cdot Schlauchquerschnittsfläche\ im\ Schlauchabschnitt\ (3)}$$

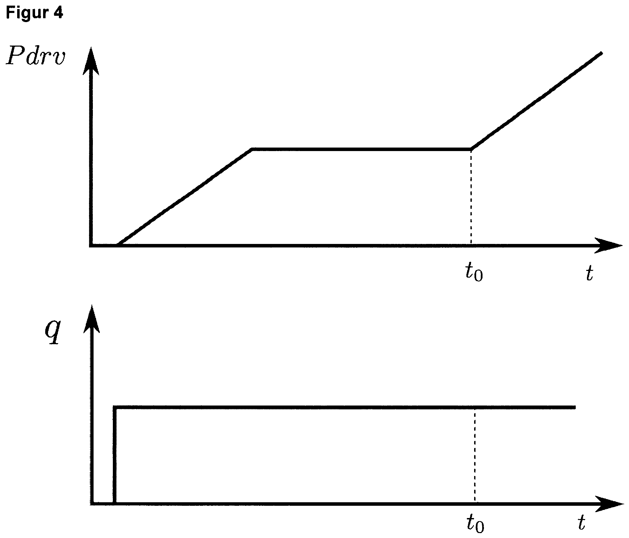

Im **Fall I) b)** und **Fall II) b)** können die Methoden aus Fall I) a) und Fall II) a) modifiziert genutzt werden. Zunächst wird der Schlauchabschnitt (7) zwischen Pumpe (5) und Patient (10) und das Instrument (9) über die Pumpe (5) mit einem Fluid gefüllt, bis der Druck am Sensor (6) stationär wird. Wird das Instrument (9) in eine geschlossene Kavität im Patienten (10) eingeführt (siehe **Figur 4,** Zeitpunkt to), steigt der Druck weiter an und wird nicht auf einen stationären Wert einlaufen, solange die Pumpe (5) ein Fluid fördert (Figur 4, Kurve mit Flow (Volumenstrom) q = konstant). Aus diesem detektierten Drucksignalverlauf lässt sich schlussfolgern, dass Fall I) b) und Fall II) b) vorliegt. In diesem Fall wird als hydrostatischer Druck $P_{wat}$ der Druckwert am Sensor (6) zum Zeitpunkt to zugewiesen.

Sollte ein **unidirektionaler Drucksensor** genutzt werden, muss die Pumpe am Zeitpunkt to gestoppt werden und das oben unter Fall **II),** a) beschriebene Verfahren durchgeführt werden, um den hydrostatischen Druck $P_{wat}$ zu berechnen.

Im **Fall I) c)** und **Fall II) c)** können erstmal keine Werte ermittelt werden. Vor dem Einsatz des Gerätes und damit vor der Notwendigkeit eine Höhenermittlung zur Kompensation zu nutzen, wird eine Situation nach dem Fall a) auftreten, weil der Anwender sich vergewissert, dass der Schlauch gefüllt ist und entsprechend die Höhenermittlung erfolgt. Es wird davon ausgegangen, dass der Fall b) im Nachgang eines Fall c) bei normaler Verwendung nicht auftreten kann. Tritt dieser dennoch auf, wird als Höhendifferenz $h_{pat}$ der Startwert bei einem Druckabfall genutzt.

**Ausführungsbeispiele**

Das erfindungsgemäße Verfahren zur Bestimmung der Höhendifferenz $h_{pat}$ zwischen Patient (10) und Fluid-Förderpumpe (5) ist wie folgt:

Ausgangssituation ist die Bereitstellung der Fluid-Förderpumpe (5) mit einem darüber angeordneten Vorratsbehälter (1), wobei die fluidischen Verbindungswege in den Vorrichtungen entsprechend zur Anwendung platziert sind, jedoch noch ungefüllt sind.

Die Pump-Vorrichtung wurde eingeschaltet und ist betriebsbereit, der Selbsttest ist abgeschlossen.

In einem ersten Schritt fördert die Fluid-Förderpumpe (5) über die durchgängige fluidische Verbindung von dem Vorratsbehälter (1) das Fluid und stoppt die Förderung, sobald der Drucksensor (6) eine Druckänderung bemerkt, welche in dieser Situation durch die geförderte Wassersäule hervorgerufen wird. Damit ist durch den Drucksensor-Messwert unter der Voraussetzung eines leeren Schlauches (3, 7) die Situation bestimmbar, dass die Fluidsäule vom Vorratsbehälter (1) zur Fluid-Förderpumpe (5) reicht und der Rest mit Luft gefüllt ist.

Der erwähnte Stopp der Förderung besteht - sofern ein Drucksensor (11) hydraulisch kurz vor bzw. oberhalb dem Förderrollenrad (4) angebracht ist - darin, den an diesem optionalen Drucksensor (11) gemessenen Wert für die weitere Verarbeitung festzuhalten und muss nicht einen Stillstand des Förderrollenrades (4) bedeuten.

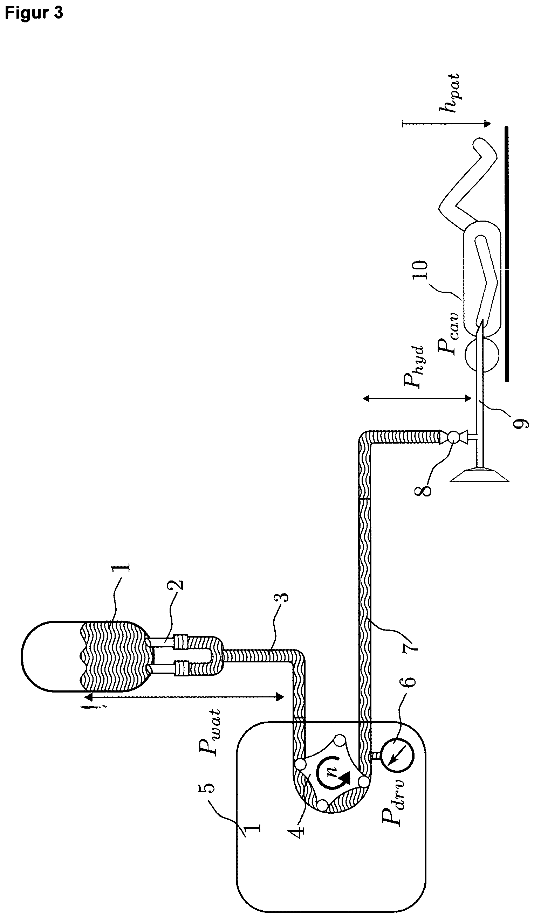

Ist kein weiterer, als der in den Figuren 1 bis 3 eingezeichnete Drucksensor (6) verfügbar, so wird das Förderrollenrad (4) rückwärts gedreht. Über eine Messung des zur Förderung aus dem Schlauchabschnitt (3) zum Vorratsbehälter (1) (also zur Rückwärtsförderung) benötigten Motorstromes wird auf das erzeugte Drehmoment geschlossen. Das Drehmoment wird verrechnet mit dem Förderrollenrad-Durchmesser als Kraft und zusammen mit der vorbekannten wirksamen Schlauchquerschnittfläche als Druckwert (Kraft / Fläche) berechnet woraus sich die Höhe zwischen dem Fluidspiegel im Vorratsbehälter (1) und der hydraulisch wirksamen Höhe des Förderrollenrades (4) ergibt.

Zwischen der hydraulisch wirksamen Höhe am Förderrollenrades (4) hin zum Vorratsbehälter (1) und der hydraulisch wirksamen Höhe am Förderrollenrades (4) hin zum Patient (10) besteht ggf. ein Unterschied, der vorab zu ermitteln ist.

Nachdem der Druck $P_{wat}$ bzw. der Höhenwert zwischen Fluidspiegel im Vorratsbehälter (1) und Fluid-Förderpumpe (5) ermittelt ist, wird die Schlauch- und Instrumentenfüllung fortgesetzt, d.h. die Pumpe (5) fördert weiter Fluid in Richtung zum Patienten (10). Die Förderung läuft bis zur kompletten Füllung des Schlauchabschnittes (7). Wenn das Fluid aus dem offenen Ende des Schlauchabschnittes (7) bzw. Instrument (9) austritt, was erkannt wird am oben beschriebenen Nachlassen des Druckanstieges wird die Füllphase/Förderung beendet. Konkret bedeutet dies, die Pumpe fördert ab diesem Zeitpunkt mit konstantem Volumenstrom, woraufhin sich ein konstanter Druckwert am Drucksensor (6) einstellt und der Druckverlauf in Relation zum geförderten Volumen ausgewertet wird. In einer alternativen Ausführungsform wird das geförderte Volumen ausgewertet und das geförderte Volumen wird bestimmt aus der Umdrehung des Förderrollenrades (4) und dem vorbekannten Fördervolumen je Umdrehung.

Die Fluid-Förderpumpe (5) wird gestoppt und der Druckwert des Drucksensors (6) ausgewertet. Ist der Druckwert positiv, wird dieser Druckwert benutzt um die Höhe zwischen Förderrollenrad (4) und Patient (10) zu bestimmen und dadurch wird davon ausgegangen der Fall liegt vor, dass die Pumpe (5) unterhalb der Höhe des Patienten (10) steht.

Ist der Druckwert negativ, wird der gemessene Druckwert als $P_{hyd}$ benutzt bzw. daraus die Höhendifferenz $h_{pat}$ ermittelt und dadurch wird davon ausgegangen der Fall liegt vor, dass die Pumpe (5) oberhalb der Höhe des Patienten (10) ist.

Ist der Druckwert 0 (null), so wird davon ausgegangen der Fall liegt vor, dass der Drucksensor (6) keine negativen Werte messen kann und die Pumpe (5) oberhalb der Höhe des Patienten (10) ist. Ist dies der Fall wird das Förderrollenrad (4) solange rückwärts gedreht und der zur Förderung aus dem Schlauchabschnitt (7) zum Vorratsbehälter (1) (also zur Rückwärtsförderung) benötigte Motorstrom gemessen und daraus auf das erzeugte Drehmoment geschlossen. Das Drehmoment wird verrechnet mit dem Förderrollenrad-Durchmesser als Kraft und zusammen mit der vorbekannten wirksamen Schlauchquerschnittfläche als Druckwert (Kraft / Fläche) berechnet woraus sich die Höhe zwischen Förderrollenrad (4) und der Höhe des Patienten (10) ergibt.

Läuft der Druckwert nach einer vorbestimmten Zeit nicht auf einen stationären Wert ein, so wird davon ausgegangen der Fall liegt vor, dass das Auslaufende in der Kavität platziert ist. Die vorbestimmte Zeit ergibt sich aus der durch die Förderrate und Schlauchquerschnittsfläche bestimmten Schlauchfüllungsdauer plus optional einem kleinen Zuschlag für das Ausspülen von Luftblasen. Im Falle das Auslaufende ist bereits in der Kavität, so ist erfindungsgemäß der Druckanstieg auszuwerten um die Patientenhöhe $h_{pat}$ zu ermitteln. Die Auswertung besteht darin, den Verlauf wie beispielhaft in Figur 4 dargestellt zu erkennen und den Wert zum Zeitpunkt to für den hydrostatischen Druck $P_{wat}$ zu übernehmen bzw. daraus abgeleitet die Höhe $h_{pat}$ zwischen Pumpe (5) und Patient (10) zu bestimmen. Hierbei ist es notwendig, dass ein konstanter Volumenstrom des Fluids erzeugt wird.

Offenbart ist ebenfalls ein Test, ob es ein offenes Ende des Schlauchabschnittes (7) bzw. Instrumentes (9) gibt, da ein Konnektor mit Auslaufventil (8) vorhanden ist, der geschlossen sein kann, sowie Klemmen am Schlauch angebracht werden können. Dieses Verfahren betrachtet den Druckverlauf zusammen mit dem geförderten Fluidvolumen. Wird ein Volumenstrom gefördert und der Druck läuft auf einen konstanten Wert am Drucksensor (6) ein, so wird davon ausgegangen der Fall liegt vor, dass der Schlauchabschnitt (7) offen zum Umgebungsdruck ist. Wird die Förderung gestoppt, baut sich der Druck ab und am Drucksensor (6) sinkt der Messwert. Sind diese Messwerte bei Flow- und DruckVerlauf vorhanden, so wird davon ausgegangen der Fall liegt vor, dass das Ende des Schlauchabschnittes (7) offen zum Umgebungsdruck ist.

Sind die Flowwerte Null und der Druck bleibt stabil bzw. sinkt nur sehr gering, so wird davon ausgegangen der Fall liegt vor, dass das Ende des Schlauchabschnittes (7) verschlossen zum Umgebungsdruck ist. Im Fall, dass die Schlauchfüllung gerade beendet ist, wird davon ausgegangen der Fall liegt vor, dass das Auslaufventil am Konnektor (8) geschlossen ist oder eine Klemme den Schlauchabschnitt (7) abklemmt. Ist dieser Fall gegeben, wird im weiteren Verlauf der Nutzung der Pumpe (5) ein Anwender die Schlauchfüllung prüfen, was bedeutet, das Auslaufventil des Konnektors (8) wird kurz auf und zu gemacht und etwas Fluid auslaufen gelassen. Der Drucksensor (6) erfasst den Druckabfall durch das Öffnen des Auslaufventiles am Konnektor (8), woraufhin bestimmungsgemäß die Förderung der Fluid-Förderpumpe (5) startet, um den voreingestellten Druck aufrechtzuerhalten. Da die Fluid-Förderpumpe (5) die Situation erkannt hat, wird der Druckwert des hydrostatischen Druckes wie bereits beschrieben durch den Drucksensor (6) oder das Rückwärtsfördern ermittelt und quasi rückwirkend der Druckwert zu Start der Förderung bzw. bei noch geschlossenem Ventil (8) als Wert $P_{wat}$ übernommen bzw. daraus die Patientenhöhe $h_{pat}$ ermittelt.

**Bezugszeichenliste**

| | |
|---|---|
| Auslaufende | bezeichnet den Übergang der nach dem Förderrollenrad (4) vorhandenen Fluidleitung zum Umgebungsdruck oder zur Kavität. Dies kann das Ende des Schlauchabschnittes (7) oder das Ende des Auslaufventils (8) oder das Ende des Instrumentes (9) sein, je nachdem wo der Umgebungsdruck bei der Anwendung der erfindungsgemäßen Methode anliegt. |
| $h_{pat}$- | Patientenhöhe = hydraulisch wirksame Höhe zwischen Förderrollenrad (4) und Patient (10) |
| P - | Druck |
| $P_m$- | Ansaugdruck |
| $P_{cav}$ - | Kavitätsdruck |
| $P_{drv}$ - | Förderdruck |
| $P_{hyd}$ - | hydrostatischer Druck des Fluides im Schlauch |
| (1) | Vorratsbehälter, Reservoir des Fluides |
| (2) | Spikes, die eine Verbindung zwischen Vorratsbehälter und Schlauch (3) bilden. Üblicherweise können hier Klemmvorrichtungen für einen Behälterwechsel angebracht sein. |
| (3) | Schlauchabschnitt des Schlauchsets zwischen Vorratsbehälter (1) bzw. Spikes (2) und Förderrollenrad (4), auch Fluidleitung |
| (4) | Förderrollenrad |
| (5) | Fluid-Förderpumpe bzw. Pumpvorrichtung oder Pumpe |
| (6) | Drucksensor - Ausführung bidirektional (misst Druck auch negativ zum Umgebungsdruck) oder unidirektional (misst nur über dem Umgebungsdruck liegende Druckwerte) |

(7)  Schlauchabschnitt zwischen Fluid-Förderpumpe (5) und Anschluss an Konnektor (8) bzw. Instrument (9), auch Fluidleitung

(8)  Konnektor mit Auslaufventil, optional vorhanden

(9)  Instrument welches in die auszudehnende Kavität eingeführt wird und aus dessen Ende das geförderte Fluid in die Kavität austritt. Häufig eine Kombination aus Hülse (= Trokar) und Endoskop oder Shaver.

(10)  Patient = Operationsgebiet = Operationsbereich = Kavität = Körperhöhle

(11)  optionaler Drucksensor, hydraulisch kurz oberhalb der Förderrollenrades (4)

(12)  Stromsensor an dem Motor, der das Förderrollenrad (4) der Fluid-Förderpumpe (5) bewegt (nicht in den Figuren dargestellt)

**Patentansprüche**

1. Verfahren zur Bestimmung der Höhendifferenz $h_{pat}$ zwischen Patient (10) und Fluid-Förderpumpe (5) mit einem Stromsensor (12) und mit einem bidirektionalen Drucksensor (6) im Schlauchabschnitt (7) zwischen Fluid-Förderpumpe (5) und Anschluss an Konnektor (8) bzw. Instrument (9),
mit einem über der Fluid-Förderpumpe (5) angeordneten Vorratsbehälter (1) einer medizintechnischen Vorrichtung, wobei die fluidischen Verbindungswege in den Vorrichtungen entsprechend zur Anwendung platziert sind, jedoch noch ungefüllt sind und wobei das Volumen der fluidischen Verbindungswege bekannt ist, **gekennzeichnet durch** folgende Verfahrensschritte:

a) Fluidförderung durch die Fluid-Förderpumpe (5) mit Förderrollenrad (4) über die durchgängige fluidische Verbindung von dem Vorratsbehälter (1)
b) Förderstopp, sobald der Drucksensor (6) eine Druckänderung bemerkt, welche durch die geförderte Wassersäule hervorgerufen wird,
c) Rückwärtsdrehung des Förderrollenrades (4) unter Messung des zur Förderung aus dem Schlauchabschnitt (3) zum Vorratsbehälter (1) benötigten Motorstromes durch den Stromsensor (12),
d) Berechnung des Druckwertes $P_{wat}$ am Patienten aus dem benötigten Motorstrom,
e) Fortsetzung der Schlauch- und Instrumentenfüllung bis zur kompletten Füllung des Schlauchabschnittes (7),
f) Erkennung des Fluidaustritts aus dem offenen Ende des Schlauchabschnittes (7) bzw. Instrumentes (9) durch Auswertung des Druckverlaufes am Drucksensor (6)
g) Beendigung der Füllphase/Förderung,
h) Auswertung des Druckwertes $P_{hyd}$ des Drucksensors (6),
i) Nutzung des Druckwertes $P_{hyd}$ zur Bestimmung der Höhendifferenz $h_{pat}$ zwischen Förderrollenrad (4) und Patient (10),

wobei

ein positiver Druckwert $P_{hyd}$ anzeigt, dass, dass die Pumpe (5) unterhalb der Höhe des Patienten (10) ist,
ein negativer Druckwert $P_{hyd}$ anzeigt, dass die Pumpe (5) oberhalb der Höhe des Patienten (10) ist,
ein Druckwert $P_{hyd}$ 0 (null) anzeigt, dass die Pumpe (5) auf der Höhe des Patienten (10) ist.

2. Verfahren zur Bestimmung der Höhendifferenz $h_{pat}$ zwischen Patient (10) und Fluid-Förderpumpe (5) mit einem Stromsensor (12) und mit einem unidirektionalen Drucksensor (6) im Schlauchabschnitt (7) zwischen Fluid-Förderpumpe (5) und Anschluss an Konnektor (8) bzw. Instrument (9),
mit einem über der Fluid-Förderpumpe (5) angeordneten Vorratsbehälter (1) einer medizintechnischen Vorrichtung, wobei die fluidischen Verbindungswege in den Vorrichtungen entsprechend zur Anwendung platziert sind, jedoch noch ungefüllt sind und wobei das Volumen der fluidischen Verbindungswege bekannt ist, **gekennzeichnet durch** folgende Verfahrensschritte:

a) Fluidförderung durch die Fluid-Förderpumpe (5) mit Förderrollenrad (4) über die durchgängige fluidische Verbindung von dem Vorratsbehälter (1)
b) Förderstopp, sobald der Drucksensor (6) eine Druckänderung bemerkt, welche durch die geförderte Wassersäule hervorgerufen wird,
c) Rückwärtsdrehung des Förderrollenrades (4) unter Messung des zur Förderung aus dem Schlauchabschnitt (3) zum Vorratsbehälter (1) benötigten Motorstromes durch den Stromsensor (12),
d) Berechnung des Druckwertes $P_{wat}$ am Patienten aus dem benötigten Motorstrom,
e) Fortsetzung der Schlauch- und Instrumentenfüllung bis zur kompletten Füllung des Schlauchabschnittes (7),

f) Erkennung des Fluidaustritts aus dem offenen Ende des Schlauchabschnittes (7) bzw. Instrumentes (9) durch Auswertung des Druckverlaufes am Drucksensor (6)

g) Beendigung der Füllphase/Förderung,

h) Auswertung des Druckwertes $P_{hyd}$ des Drucksensors (6),

i) Nutzung des positiven Druckwertes $P_{hyd}$ zur Bestimmung der Höhendifferenz $h_{pat}$ zwischen Förderrollenrad (4) und Patient (10),

wobei

der positiver Druckwert $P_{hyd}$ anzeigt, dass die Pumpe (5) unterhalb der Höhe des Patienten (10) ist,

ein Druckwert $P_{hyd}$ 0 (null) anzeigt, dass die Pumpe (5) auf der Höhe des Patienten (10) oder oberhalb der Höhe des Patienten (10) ist;

j) im Fall eines Druckwertes $P_{hyd}$ 0 (null) Rückwärtsförderung des Fluides durch Rückwärtsdrehung des Förderrollenrades (4),

k) Bestimmung des Motorstromes durch Auswertung des Stromsensors (12) und Berechnung des Druckes zur Bestimmung der Höhe zwischen Förderrollenrad (4) und der Höhe des Patienten (10), wobei die Pumpe (5) unterhalb der Höhe des Patienten (10) ist.

3. Verfahren zur Bestimmung der Höhendifferenz $h_{pat}$ zwischen Patient (10) und Fluid-Förderpumpe (5) mit einem Stromsensor (12) und mit einem bidirektionalen Drucksensor (6) im Schlauchabschnitt (7) zwischen Fluid-Förderpumpe (5) und Anschluss an Konnektor (8) bzw. Instrument (9) sowie mit einem weiteren Drucksensor 11 zwischen einem Vorratsbehälter (1) und dem Förderrollenrad (4) mit einem über der Fluid-Förderpumpe (5) angeordneten Vorratsbehälter (1) einer medizintechnischen Vorrichtung, wobei die fluidischen Verbindungswege in den Vorrichtungen entsprechend zur Anwendung platziert sind, jedoch noch ungefüllt sind und wobei das Volumen der fluidischen Verbindungswege bekannt ist, **gekennzeichnet durch** folgende Verfahrensschritte:

a) Fluidförderung durch die Fluid-Förderpumpe (5) mit Förderrollenrad (4) über die durchgängige fluidische Verbindung von dem Vorratsbehälter (1) zum Rollenrad,

b) Förderstopp, sobald der Drucksensor (6) eine Druckänderung bemerkt, welche durch die geförderte Wassersäule hervorgerufen wird,

c) Ablesen des Druckwertes $P_{wat}$ am Drucksensor 11,

d) Fortsetzung der Schlauch- und Instrumentenfüllung bis zur kompletten Füllung des Schlauchabschnittes (7),

e) Erkennung des Fluidaustritts aus dem offenen Ende des Schlauchabschnittes (7) bzw. Instrumentes (9) durch Auswertung des Druckverlaufes am Drucksensor (6)

f) Beendigung der Füllphase/Förderung,

g) Auswertung des Druckwertes $P_{hyd}$ des Drucksensors (6),

h) Nutzung des Druckwertes $P_{hyd}$ zur Bestimmung der Höhendifferenz $h_{pat}$ zwischen Förderrollenrad (4) und Patient (10),

wobei

ein positiver Druckwert $P_{hyd}$ anzeigt, dass, dass die Pumpe (5) unterhalb der Höhe des Patienten (10) ist,

ein negativer Druckwert $P_{hyd}$ anzeigt, dass die Pumpe (5) oberhalb der Höhe des Patienten (10) ist,

ein Druckwert $P_{hyd}$ 0 (null) anzeigt, dass die Pumpe (5) auf der Höhe des Patienten (10) ist.

4. Verfahren zur Bestimmung der Höhendifferenz $h_{pat}$ zwischen Patient (10) und Fluid-Förderpumpe (5) mit einem Stromsensor (12) und mit einem unidirektionalen Drucksensor (6) im Schlauchabschnitt (7) zwischen Fluid-Förderpumpe (5) und Anschluss an Konnektor (8) bzw. Instrument (9) sowie mit einem weiteren Drucksensor 11 zwischen einem Vorratsbehälter (1) und dem Förderrollenrad (4),

mit einem über der Fluid-Förderpumpe (5) angeordneten Vorratsbehälter (1) einer medizintechnischen Vorrichtung, wobei die fluidischen Verbindungswege in den Vorrichtungen entsprechend zur Anwendung platziert sind, jedoch noch ungefüllt sind und wobei das Volumen der fluidischen Verbindungswege bekannt ist, **gekennzeichnet durch** folgende Verfahrensschritte:

a) Fluidförderung durch die Fluid-Förderpumpe (5) mit Förderrollenrad (4) über die durchgängige fluidische Verbindung von dem Vorratsbehälter (1)

b) Förderstopp, sobald der Drucksensor (6) eine Druckänderung bemerkt, welche durch die geförderte Wassersäule hervorgerufen wird,

c) Ablesen des Druckwertes $P_{wat}$ am Drucksensor 11,

d) Fortsetzung der Schlauch- und Instrumentenfüllung bis zur kompletten Füllung des Schlauchabschnittes (7),

e) Erkennung des Fluidaustritts aus dem offenen Ende des Schlauchabschnittes (7) bzw. Instrumentes (9) durch Auswertung des Druckverlaufes am Drucksensor (6)

f) Beendigung der Füllphase/Förderung,

g) Auswertung des Druckwertes $P_{hyd}$ des Drucksensors (6),

h) Nutzung des positiven Druckwertes $P_{hyd}$ zur Bestimmung der Höhendifferenz $h_{pat}$ zwischen Förderrollenrad (4) und Patient (10),

wobei

der positiver Druckwert $P_{hyd}$ anzeigt, dass, dass die Pumpe (5) unterhalb der Höhe des Patienten (10) ist, ein Druckwert $P_{hyd}$ 0 (null) anzeigt, dass die Pumpe (5) auf der Höhe des Patienten (10) oder oberhalb der Höhe des Patienten (10) ist;

i) im Fall eines Druckwertes $P_{hyd}$ 0 (null) Rückwärtsförderung des Fluides durch Rückwärtsdrehung des Förderrollenrades (4),

j) Bestimmung des Motorstromes durch Auswertung des Stromsensors (12) und Berechnung des Druckes zur Bestimmung der Höhe zwischen Förderrollenrad (4) und der Höhe des Patienten (10), wobei die Pumpe (5) unterhalb der Höhe des Patienten (10) ist.

5. Verfahren gemäß einem der Ansprüche 1-4, **dadurch gekennzeichnet, dass** die Erkennung des ordnungsgemäßen Austritts des Fluids durch den Förderschlauch (7) und das medizinische Instrument (9) in die Kavität des Patienten (10) durch folgende Schritte erfolgt:

a) Fluidförderung durch die Pumpe (5) bis zur Anzeige eines von null abweichenden Druckwertes am Drucksensor (6)

b) weitere Fluidförderung bis das geförderte Volumen größer ist als das Schlauchvolumen wobei der am Drucksensor (6) gemessene Druck konstant bleibt,

c) Feststellung eines Druckanstiegs am Drucksensor (6) nach Förderung eines Flüssigkeitsvolumens welches größer ist als das Schlauchvolumen.

6. Verfahren gemäß einem der Ansprüche 1-4, **dadurch gekennzeichnet, dass** die Erkennung des fehlerhaft geschlossenen Ventils (8) durch folgende Schritte erfolgt:

a) Fluidförderung durch die Pumpe (5) bis zur Anzeige eines von null abweichenden Druckwertes am Drucksensor (6)

b) weitere Fluidförderung wobei der am Drucksensor (6) gemessene Druck signifikant ansteigt bevor das geförderte Volumen dem Schlauchvolumen entspricht.

7. Verfahren gemäß einem der Ansprüche 1-4, **dadurch gekennzeichnet, dass** die Erkennung des fehlerhaft nicht in der Körperhöhle befindlichen Instrumentes oder einer nicht geschlossenen Verbindung zwischen Förderschlauch (7) und medizinischem Instrument (9) durch folgende Schritte erfolgt:

a) Fluidförderung durch die Pumpe (5) bis zur Anzeige eines von null abweichenden Druckwertes am Drucksensor (6)

b) weitere Fluidförderung bis das geförderte Volumen größer ist als das Schlauchvolumen wobei der am Drucksensor (6) gemessene Druck konstant bleibt,

c) Feststellung, dass nach Förderung eines Flüssigkeitsvolumens welches größer ist als das Schlauchvolumen kein Druckanstieg am Drucksensor (6) gemessen wird.

8. Verfahren gemäß einem der Ansprüche 1-4, **dadurch gekennzeichnet, dass** das Volumen der fluidischen Verbindungswege von der Pumpe vor Verfahrensbeginn aus einem am Schlauchset befindlichen Informationsträger ausgelesen wird.

9. Verfahren gemäß Anspruch 8, **dadurch gekennzeichnet, dass** der Informationsträger ein RFID-Chip, ein Magnetstreifen, ein Barcode oder ein EPROM ist.

**Claims**

1. A method for determining the difference in height level $h_{pat}$ between a patient (10) and a fluid feed pump (5) with a current sensor (12) and with a bidirectional pressure sensor (6) in a tube section (7) between the fluid feed pump (5) and the connection to a connector (8) or an instrument (9), comprising a reservoir (1) of a medical device arranged above the fluid feed pump (5), the fluidic connection paths provided in the devices according to the application, being, however, still unfilled, and the volume of the fluidic connection paths being known, **characterized by** the following steps:

> a) fluid feeding by the fluid feed pump (5) with a transport roller wheel (4) via the through-going fluidic connection of the reservoir (1),
> b) stop of feeding, as soon as the pressure sensor (6) detects a change in pressure, which is caused by the fed water column,
> c) backward rotation of the transport roller wheel (4) while measuring, by the current sensor (12), the motor current required for feeding from the tube section (3) to the reservoir (1),
> d) calculating the pressure value $P_{wat}$ at the patient from the required motor current,
> e) continuing the tube and instrument filling process until the tube section (7) is completely filled,
> f) detecting the fluid exit from the open end of the tube section (7) or instrument (9) by evaluating the pressure value at the pressure sensor (6),
> g) terminating the filling phase/feeding process,
> h) evaluating the pressure value $P_{hyd}$ of the pressure sensor (6),
> i) using the pressure value $P_{hyd}$ for determining the difference in height level $h_{pat}$ between the transport roller wheel (4) and the patient (10),

wherein

> a positive pressure value $P_{hyd}$ indicates that the pump (5) is below the height level of the patient (10),
> a negative pressure value $P_{hyd}$ indicates that the pump (5) is above the height level of the patient (10),
> a pressure value $P_{hyd}$ 0 (zero) indicates that the pump (5) is at the height level of the patient (10).

2. A method for determining the difference in height level $h_{pat}$ between a patient (10) and a fluid feed pump (5) with a current sensor (12) and with a unidirectional pressure sensor (6) in a tube section (7) between the fluid feed pump (5) and the connection to a connector (8) or an instrument (9), comprising a reservoir (1) of a medical device arranged above the fluid feed pump (5), the fluidic connection paths provided in the devices according to the application, being, however, still unfilled, and the volume of the fluidic connection paths being known, **characterized by** the following steps:

> a) fluid feeding by the fluid feed pump (5) with a transport roller wheel (4) via the through-going fluidic connection of the reservoir (1),
> b) stop of feeding, as soon as the pressure sensor (6) detects a change in pressure, which is caused by the fed water column,
> c) backward rotation of the transport roller wheel (4) while measuring, by the current sensor (12), the motor current required for feeding from the tube section (3) to the reservoir (1),
> d) calculating the pressure value $P_{wat}$ at the patient from the required motor current,
> e) continuing the tube and instrument filling process until the tube section (7) is completely filled,
> f) detecting the fluid exit from the open end of the tube section (7) or instrument (9) by evaluating the pressure value at the pressure sensor (6),
> g) terminating the filling phase/feeding process,
> h) evaluating the pressure value $P_{hyd}$ of the pressure sensor (6),
> i) using the positive pressure value $P_{hyd}$ for determining the difference in height level $h_{pat}$ between the transport roller wheel (4) and the patient (10),

wherein

> a positive pressure value $P_{hyd}$ indicates that the pump (5) is below the height level of the patient (10),
> a pressure value $P_{hyd}$ 0 (zero) indicates that the pump (5) is at the height level of the patient (10) or above the height level of the patient (10),
> j) in the case of a pressure value $P_{hyd}$ 0 (zero), backward feeding of the fluid by backward rotation of the transport roller wheel (4),

k) determining the motor current by evaluation of the current sensor (12) and calculating the pressure for determining the height level between the transport roller wheel (4) and the height of patient (10), the pump (5) being below the height level of the patient (10).

3. A method for determining the difference in height level $h_{pat}$ between a patient (10) and a fluid feed pump (5) with a current sensor (12) and with a bidirectional pressure sensor (6) in the tube section (7) between the fluid feed pump (5) and the connection to a connector (8) or an instrument (9), and with another pressure sensor (11) between a reservoir (1) and the transport roller wheel (4), with the reservoir (1) of a medical device being arranged above the fluid feed pump (5), the fluidic connection paths provided in the devices according to the application, being, however, still unfilled, and the volume of the fluidic connection paths being known, **characterized by** the following steps:

a) fluid feeding by the fluid feed pump (5) with a transport roller wheel (4) via the through-going fluidic connection of the reservoir (1) to the roller wheel,
b) stop of feeding, as soon as the pressure sensor (6) detects a change in pressure, which is caused by the fed water column,
e) reading the pressure value $P_{wat}$ at the pressure sensor (11),
d) continuing the tube and instrument filling process until the tube section (7) is completely filled,
e) detecting the fluid exit from the open end of the tube section (7) or instrument (9) by evaluating the pressure value at the pressure sensor (6)
f) terminating the filling phase/feeding process,
g) evaluating the pressure value $P_{hyd}$ of the pressure sensor (6),
h) using the pressure value $P_{hyd}$ for determining the difference in height level $h_{pat}$ between the transport roller wheel (4) and the patient (10),

wherein

a positive pressure value $P_{hyd}$ indicates that the pump (5) is below the height level of the patient (10),
a negative pressure value $P_{hyd}$ indicates that the pump (5) is above the height level of the patient (10),
a pressure value $P_{hyd}$ 0 (zero) indicates that the pump (5) is at the height level of the patient (10).

4. A method for determining the difference in height level $h_{pat}$ between a patient (10) and a fluid feed pump (5) with a current sensor (12) and with a unidirectional pressure sensor (6) in the tube section (7) between the fluid feed pump (5) and the connection to a connector (8) or an instrument (9), and with another pressure sensor (11) between a reservoir (1) and the transport roller wheel (4), with the reservoir (1) of a medical device being arranged above the fluid feed pump (5), the fluidic connection paths provided in the devices according to the application, being, however, still unfilled, and the volume of the fluidic connection paths being known, **characterized by** the following steps:

a) fluid feeding by the fluid feed pump (5) with a transport roller wheel (4) via the through-going fluidic connection of the reservoir (1),
b) stop of feeding, as soon as the pressure sensor (6) detects a change in pressure, which is caused by the fed water column,
c) reading the pressure value $P_{wat}$ at the pressure sensor (11),
d) continuing the tube and instrument filling process until the tube section (7) is completely filled,
e) detecting the fluid exit from the open end of the tube section (7) or instrument (9) by evaluating the pressure value at the pressure sensor (6),
f) terminating the filling phase/feeding process,
g) evaluating the pressure value $P_{hyd}$ of the pressure sensor (6),
h) using the positive pressure value $P_{hyd}$ for determining the difference in height level $h_{pat}$ between the transport roller wheel (4) and the patient (10),

wherein

a positive pressure value $P_{hyd}$ indicates that the pump (5) is below the height level of the patient (10),
a pressure value $P_{hyd}$ 0 (zero) indicates that the pump (5) is at the height level of the patient (10) or above the height level of the patient (10),

i) in the case of a pressure value $P_{hyd}$ 0 (zero), backward feeding of the fluid by backward rotation of the transport roller wheel (4),

j) determining the motor current by evaluation of the current sensor (12) and calculating the pressure for determining the height level between the transport roller wheel (4) and the patient (10), the pump (5) being below the height level of the patient (10).

5. The method according to one of claims 1 to 4, **characterized by** that the detection of the proper exit of the fluid through the transport tube (7) and the medical instrument (9) into the cavity of the patient (10) is performed by the following steps:

a) fluid feeding by the pump (5) until a pressure value different from zero is indicated at the pressure sensor (6),

b) further fluid feeding until the fed volume is larger than the volume of the tube, with the pressure measured at the pressure sensor (6) remaining constant,

c) detecting a pressure increase at the pressure sensor (6) after feeding a liquid volume, which is larger than the volume of the tube.

6. The method according to one of claims 1 to 4, **characterized by** that the detection of the faultily closed valve (8) is performed by the following steps:

a) fluid feeding by the pump (5) until a pressure value different from zero is indicated at the pressure sensor (6),

b) further fluid feeding, wherein the pressure measured at the pressure sensor (6) significantly increases, before the fed volume corresponds to the volume of the tube.

7. The method according to one of claims 1 to 4, **characterized by** that the detection of the instrument faultily not being in the body cavity or of a non-closed connection between transport tube (7) and medical instrument (9) is performed by the following steps:

a) fluid feeding by the pump (5) until a pressure value different from zero is indicated at the pressure sensor (6)

b) further fluid feeding until the fed volume is larger than the volume of the tube, with the pressure measured at the pressure sensor (6) remaining constant,

c) detection that after feeding a liquid volume, which is larger than the volume of the tube, no pressure increase is measured at the pressure sensor (6).

8. The method according to one of claims 1 to 4, **characterized by** that the volume of the fluidic connection paths of the pump is read out from an information carrier provided at the tube set before starting the process.

9. The method according to claim 8,
**characterized by** that the information carrier is an RFID-Chip, a magnetic tape, a barcode, or an EPROM.

**Revendications**

1. Procédé de détermination de la différence en hauteur $h_{pat}$ entre un patient (10) et une pompe de refoulement de fluide (5) avec un capteur de courant (12) et avec un capteur de pression (6) bidirectionnel dans une portion de tube (7) entre la pompe de refoulement de fluide (5) et le raccordement à un raccord (8) ou un instrument (9), comprenant un réservoir (1) d'un dispositif médical disposé au-dessus de la pompe de refoulement de fluide (5), les chemins de raccordement fluidiques étant positionnés dans les dispositifs selon l'application, étant, cependant, encore non remplis, et le volume des chemins de raccordement fluidiques étant connu,
**caractérisé par** les étapes suivantes:

a) refoulement de fluide par la pompe de refoulement de fluide (5) avec une roue à galets de refoulement (4) par l'intermédiaire du raccordement fluidique traversant du réservoir (1),

b) arrêt de refoulement, dès que le capteur de pression (6) détecte une variation de pression, qui est causée par la colonne d'eau refoulée,

c) rotation de la roue à galets de refoulement (4) en arrière pendant que le courant du moteur nécessaire pour le refoulement à partir de la portion de tube (3) au réservoir (1) est mesuré par le capteur de courant (12),

d) calcul de la pression $P_{wat}$ au patient à partir du courant du moteur nécessaire,

e) continuation du procédé de remplissage du tube et de l'instrument, jusqu'à ce que la portion de tube (7) soit complètement remplie,

f) détection de la sortie de fluide à partir de l'extrémité ouverte de la portion de tube (7) ou de l'instrument (9) par évaluation de la pression au capteur de pression (6),

g) arrêt de la phase de remplissage/du procédé de refoulement,

h) évaluation de la pression $P_{hyd}$ du capteur de pression (6),

i) utilisation de la pression $P_{hyd}$ pour déterminer la différence en hauteur $h_{pat}$ entre la roue à galets de refoulement (4) et le patient (10),

dans lequel

une pression $P_{hyd}$ positive indique que la pompe (5) est au-dessous du niveau de hauteur du patient (10),

une pression $P_{hyd}$ négative indique que la pompe (5) est au-dessus du niveau de hauteur du patient (10),

une pression $P_{hyd}$ 0 (zéro) indique que la pompe (5) est au niveau de hauteur du patient (10).

2. Procédé de détermination de la différence en hauteur $h_{pat}$ entre un patient (10) et une pompe de refoulement de fluide (5) avec un capteur de courant (12) et avec un capteur de pression (6) unidirectionnel dans une portion de tube (7) entre la pompe de refoulement de fluide (5) et le raccordement à un raccord (8) ou un instrument (9), comprenant un réservoir (1) d'un dispositif médical disposé au-dessus de la pompe de refoulement de fluide (5), les chemins de raccordement fluidiques étant positionnés dans les dispositifs selon l'application, étant, cependant, encore non remplis, et le volume des chemins de raccordement fluidiques étant connu, **caractérisé par** les étapes suivantes:

a) refoulement de fluide par la pompe de refoulement de fluide (5) avec une roue à galets de refoulement (4) par l'intermédiaire du raccordement fluidique traversant du réservoir (1),

b) arrêt de refoulement, dès que le capteur de pression (6) détecte une variation de pression, qui est causée par la colonne d'eau refoulée,

c) rotation de la roue à galets de refoulement (4) en arrière pendant que le courant du moteur nécessaire pour le refoulement à partir de la portion de tube (3) au réservoir (1) est mesuré par le capteur de courant (12),

d) calcul de la pression $P_{wat}$ au patient à partir du courant du moteur nécessaire,

e) continuation du procédé de remplissage du tube et de l'instrument, jusqu'à ce que la portion de tube (7) soit complètement remplie,

f) détection de la sortie de fluide à partir de l'extrémité ouverte de la portion de tube (7) ou de l'instrument (9) par évaluation de la pression au capteur de pression (6),

g) arrêt de la phase de remplissage/du procédé de refoulement,

h) évaluation de la pression $P_{hyd}$ du capteur de pression (6),

i) utilisation de la pression $P_{hyd}$ positive pour déterminer la différence en hauteur $h_{pat}$ entre la roue à galets de refoulement (4) et le patient (10),

dans lequel

une pression $P_{hyd}$ positive indique que la pompe (5) est au-dessous du niveau de hauteur du patient (10),

une pression $P_{hyd}$ 0 (zéro) indique que la pompe (5) est au niveau de hauteur du patient (10) ou au-dessus du niveau de hauteur du patient (10),

j) dans le cas d'une pression $P_{hyd}$ 0 (zéro), refoulement du fluide en arrière par rotation de la roue à galets de refoulement (4) en arrière,

k) détermination du courant du moteur par évaluation du capteur de courant (12) et calcul de la pression pour déterminer le niveau de hauteur entre la roue à galets de refoulement (4) et le patient (10), la pompe (5) étant au-dessous du niveau de hauteur du patient (10).

3. Procédé de détermination de la différence en hauteur $h_{pat}$ entre un patient (10) et une pompe de refoulement de fluide (5) avec un capteur de courant (12) et avec un capteur de pression (6) bidirectionnel dans la portion de tube (7) entre la pompe de refoulement de fluide (5) et le raccordement à un raccord (8) ou un instrument (9), et avec un autre capteur de pression (11) entre un réservoir (1) et la roue à galets de refoulement (4), le réservoir (1) d'un dispositif médical étant disposé au-dessus de la pompe de refoulement de fluide (5), les chemins de raccordement fluidiques étant positionnés dans les dispositifs selon l'application, étant, cependant, encore non remplis, et le volume des chemins de raccordement fluidiques étant connu, **caractérisé par** les étapes suivantes:

a) refoulement de fluide par la pompe de refoulement de fluide (5) avec une roue à galets de refoulement (4) par l'intermédiaire du raccordement fluidique traversant du réservoir (1) à la roue à galets,

b) arrêt de refoulement, dès que le capteur de pression (6) détecte une variation de pression, qui est causée par la colonne d'eau refoulée,

e) lecture de la pression $P_{wat}$ au capteur de pression (11),

d) continuation du procédé de remplissage du tube et de l'instrument, jusqu'à ce que la portion de tube (7) soit complètement remplie,

e) détection de la sortie de fluide à partir de l'extrémité ouverte de la portion de tube (7) ou de l'instrument (9) par évaluation de la pression au capteur de pression (6)

f) arrêt de la phase de remplissage/du procédé de refoulement,

g) évaluation de la pression $P_{hyd}$ du capteur de pression (6),

h) utilisation de la pression $P_{hyd}$ pour déterminer la différence en hauteur $h_{pat}$ entre la roue à galets de refoulement (4) et le patient (10),

dans lequel

une pression $P_{hyd}$ positive indique que la pompe (5) est au-dessous du niveau de hauteur du patient (10),
une pression $P_{hyd}$ négative indique que la pompe (5) est au-dessus du niveau de hauteur du patient (10),
une pression $P_{hyd}$ 0 (zéro) indique que la pompe (5) est au niveau de hauteur du patient (10).

4. Procédé de détermination de la différence en hauteur $h_{pat}$ entre un patient (10) et une pompe de refoulement de fluide (5) avec un capteur de courant (12) et avec un capteur de pression (6) unidirectionnel dans la portion de tube (7) entre la pompe de refoulement de fluide (5) et le raccordement à un raccord (8) ou un instrument (9), et avec un autre capteur de pression (11) entre un réservoir (1) et la roue à galets de refoulement (4), le réservoir (1) d'un dispositif médical étant disposé au-dessus de la pompe de refoulement de fluide (5), les chemins de raccordement fluidiques étant positionnés dans les dispositifs selon l'application, étant, cependant, encore non remplis, et le volume des chemins de raccordement fluidiques étant connu, **caractérisé par** les étapes suivantes:

a) refoulement de fluide par la pompe de refoulement de fluide (5) avec une roue à galets de refoulement (4) par l'intermédiaire du raccordement fluidique traversant du réservoir (1),

b) arrêt de refoulement, dès que le capteur de pression (6) détecte une variation de pression, qui est causée par la colonne d'eau refoulée,

c) lecture de la pression $P_{wat}$ au capteur de pression (11),

d) continuation du procédé de remplissage du tube et de l'instrument, jusqu'à ce que la portion de tube (7) soit complètement remplie,

e) détection de la sortie de fluide à partir de l'extrémité ouverte de la portion de tube (7) ou de l'instrument (9) par évaluation de la pression au capteur de pression (6),

f) arrêt de la phase de remplissage/du procédé de refoulement,

g) évaluation de la pression $P_{hyd}$ du capteur de pression (6),

h) utilisation de la pression $P_{hyd}$ positive pour déterminer la différence en hauteur $h_{pat}$ entre la roue à galets de refoulement (4) et le patient (10),

dans lequel

une pression $P_{hyd}$ positive indique que la pompe (5) est au-dessous du niveau de hauteur du patient (10),
une pression $P_{hyd}$ 0 (zéro) indique que la pompe (5) est au niveau de hauteur du patient (10) ou au-dessus du niveau de hauteur du patient (10),

i) dans le cas d'une pression $P_{hyd}$ 0 (zéro), refoulement du fluide en arrière par rotation de la roue à galets de refoulement (4) en arrière,

j) détermination du courant du moteur par évaluation du capteur de courant (12) et calcul de la pression pour déterminer le niveau de hauteur entre la roue à galets de refoulement (4) et le patient (10), la pompe (5) étant au-dessous du niveau de hauteur du patient (10).

5. Procédé selon une de revendications 1 à 4, **caractérisé en ce que** la détection de la sortie appropriée du fluide à travers le tube de refoulement (7) et l'instrument médical (9) dans la cavité du patient (10) a lieu par les étapes suivantes:

a) refoulement de fluide par la pompe (5), jusqu'à ce qu'une pression non zéro soit indiquée au capteur de pression (6),
b) continuation de refoulement de fluide, jusqu'à ce que le volume refoulé soit supérieur au volume du tube, la pression mesurée au capteur de pression (6) restant constante,
c) détection d'une montée en pression au capteur de pression (6) après refoulement d'un volume liquide, qui est supérieur au volume du tube.

6. Procédé selon une de revendications 1 à 4, **caractérisé en ce que** la détection de la vanne (8) fermée de manière incorrecte a lieu par les étapes suivantes :

a) refoulement de fluide par la pompe (5), jusqu'à ce qu'une pression non zéro soit indiquée au capteur de pression (6),
b) continuation de refoulement de fluide, la pression mesurée au capteur de pression (6) sensiblement montant, avant que le volume refoulé corresponde au volume du tube.

7. Procédé selon une de revendications 1 à 4, **caractérisé en ce que** la détection de l'instrument ne se trouvant pas de manière incorrecte dans la cavité du corps ou d'un raccordement non fermé entre le tube de refoulement (7) et l'instrument médical (9) a lieu par les étapes suivantes:

a) refoulement de fluide par la pompe (5), jusqu'à ce qu'une pression non zéro soit indiquée au capteur de pression (6)
b) continuation de refoulement de fluide, jusqu'à ce que le volume refoulé soit supérieur au volume du tube, la pression mesurée au capteur de pression (6) restant constante,
c) détection qu'après le refoulement d'un volume liquide, qui est supérieur au volume du tube, aucune montée en pression n'est mesurée au capteur de pression (6).

8. Procédé selon une de revendications 1 à 4, **caractérisé en ce que** le volume des chemins de raccordement fluidiques de la pompe est lu à partir d'un support d'information prévu au jeu des tubes avant le départ du procédé.

9. Procédé selon la revendication 8, **caractérisé en ce que** le support d'information est une puce RFID, une bande magnétique, un code à barre ou une EPROM.

**Figur 1**

**Figur 2**

**Figur 3**

**Figur 4**

$Pdrv$

$t_0$

$t$

$q$

$t_0$

$t$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20150290387 A1
- US 20080243054 A1
- WO 2005089832 A2
- WO 2005042065 A2
- US 2017348137 A1
- US 2017209639 A1
- US 4795424 A